# EUROPEAN PATENT APPLICATION

(11) **EP 3 539 950 A1**
(43) Date of publication of application: **18.09.2019**
(21) Application number: 19160299.4
(22) Date of filing: 01.03.2019
(51) Int. Cl.: C07D 209/46

(54) **3-OXO-2-ALKYLISOINDOLINE DIPHTHALONITRILE MONOMERS, METHODS OF MANUFACTURE, THERMOSETTING COMPOSITIONS COMPRISING THE SAME, AND USES THEREOF**

(30) Priority: 14.03.2018 EP 18161749
(71) Applicant: SABIC Global Technologies B.V., 4612 PX Bergen op Zoom (NL)
(72) Inventor: KONDA, SHIVAKUMAR, 562125 Bangalore, Karnataka (IN); CHOWDHURY, RAJESH, 562125 Bangalore, Karnataka (IN); POLASA, INDIRA JYOTHI, 562125 Bangalore, Karnataka (IN)
(74) Representative: Modiano, Micaela Nadia

(57) **Abstract**

A thermosetting composition, comprising a 4,4'-(((3-oxo-2-hydrocarbylisoindoline-1,1-diyl)bis(4,1-arylene))bis(oxy)) diphthalonitrile compound of formula (1) 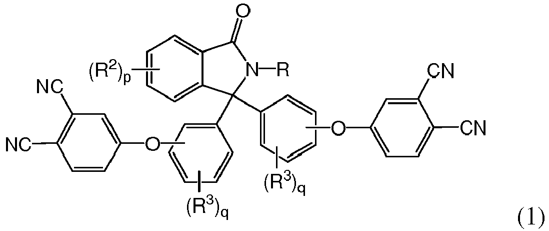 wherein R is a C₁₋₁₂ hydrocarbyl, preferably a C₁₋₆ alkyl or a C₆₋₁₂ aryl; each occurrence of R² and R³ is independently a hydrogen, a halogen, or a C₁₋₂₅ hydrocarbyl, preferably hydrogen or a C₁₋₆ alkyl, more preferably a hydrogen or C₁₋₃ alkyl; and p and q are each independently 0 to 4, preferably 0 or 1, more preferably 0.

## Description

### BACKGROUND

Phthalonitrile polymers constitute an important class of high-temperature materials, having a wide range of uses, such as composite matrices, adhesives, sealants, and semiconductors.

Phthalonitrile (PN) and diphthalonitrile (DPN) monomers can provide heat-curable crosslinked polymers having superior flammability and high temperature properties, relative to other high temperature polymers. The PN and DPN compounds polymerize through the cyano groups to provide a crosslinked polymeric network with high thermal and oxidative stability, as well as a high modulus of elasticity.

DPN compounds include a spacer group that links the moieties having the phthalonitrile functionality. Aliphatic spacer groups are readily cured compared to DPN compounds having aromatic spacer groups. The cure temperature of DPN compounds having aromatic spacers can be higher than for aliphatic-spacer linked compounds. The relatively slow rate of polymerization for aromatic spacer-linked DPN compounds has been attributed to the rigidity of the linking structure, which can reduce the mobility of the cyano groups.

There accordingly remains a need for thermoset polymer products derived from DPN monomers having aromatic bridging groups, and methods of synthesizing such thermoset polymers.

### SUMMARY

Disclosed herein is a thermosetting composition, comprising a 4,4'-(((3-oxo-2-hydrocarbylisoindoline-1,1-diyl)bis(4,1-arylene))bis(oxy)) diphthalonitrile compound of formula (1) 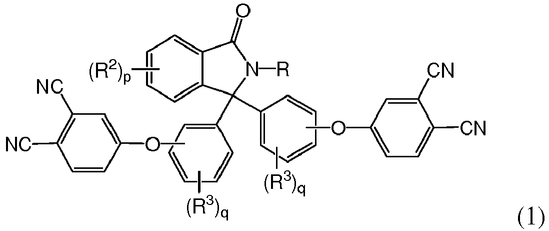 wherein R is a C₁₋₁₂ hydrocarbyl, preferably a C₁₋₆ alkyl or a C₆₋₁₂ aryl; each occurrence of R² and R³ is independently a hydrogen, a halogen, or a C₁₋₂₅ hydrocarbyl, preferably hydrogen or a C₁₋₆ alkyl, more preferably a hydrogen or C₁₋₃ alkyl; and p and q are each independently 0 to 4, preferably 0 or 1, more preferably 0.

According to another aspect, a method for the manufacture of the 4,4'-(((3-oxo-2-hydrocarbylisoindoline-1,1-diyl)bis(4,1-arylene))bis(oxy)) diphthalonitrile compound (1) comprises reacting a 3,3-bis(4-hydroxyaryl)-2-hydrocarbylisoindolin-1-one of formula (2) 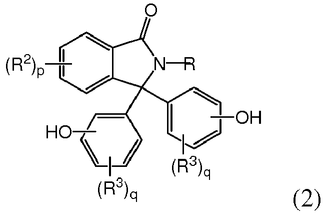 wherein R is a C₁₋₁₂ hydrocarbyl, preferably a C₁₋₆ alkyl; each occurrence of R² and R³ is independently a hydrogen, a halogen, a C₁₋₂₅ hydrocarbyl, preferably a hydrogen or a C₁₋₆ alkyl, more preferably a hydrogen or a C₁₋₃ alkyl; and p and q are each independently 0-4, preferably 0 or 1, more preferably 0, with 4-nitrophthalonitrile under conditions effective to provide the 4,4'-(((3-oxo-2-hydrocarbylisoindoline-1,1-diyl)bis(4,1-arylene))bis(oxy))diphthalonitrile compound (1).

Another aspect provides a thermoset polymer composition comprising a crosslinked product of the thermosetting composition.

An article comprises the thermoset polymer composition, wherein the article is a composite, a foam, a fiber, a layer, a coating, an encapsulant, an adhesive, a sealant, a component, a prepreg, a casing, or a combination thereof.

The disclosure is further illustrated by the following detailed description, examples, and claims.

### DETAILED DESCRIPTION

Described herein are aromatic diphthalonitrile (DPN) monomers and uses thereof. The aromatic DPN monomers have an aromatic spacer that can provide good thermo-oxidative resistance, and can be used to provide thermoset polymers with potentially well-defined melting points. For example, thermoset polymers including the aromatic spacer-linked DPN monomers can withstand high temperatures for extended periods of time without permanent damage. In particular, the resulting thermoset polymers can withstand temperatures of up 500°C, preferably 200 to 500°C, more preferably 200 to 400°C. The thermoset polymers derived from the aromatic DPN monomers can be superior in physical and chemical properties to epoxies, polyimides, and other polymers used as high temperature adhesives.

Disclosed herein is a thermosetting composition including a DPN compound, 4,4'-(((3-oxo-2-hydrocarbylisoindoline-1,1-diyl)bis(4,1-arylene))bis(oxy)) diphthalonitrile, of formula (1) 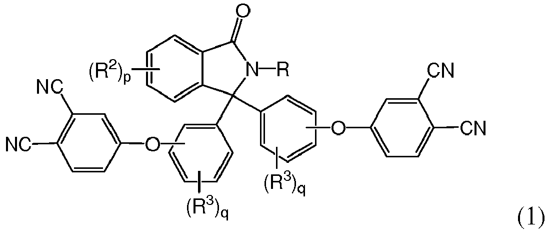 wherein R is a C₁₋₁₂ hydrocarbyl, preferably a C₁₋₆ alkyl or a C₆₋₁₂ aryl, even more preferably a C₁₋₆ alkyl. For example, R is methyl. Each occurrence of R² and R³ is independently a hydrogen, a halogen, or a C₁₋₂₅ hydrocarbyl, preferably hydrogen or a C₁₋₆ alkyl, more preferably a hydrogen or C₁₋₃ alkyl. In formula (1), p and q are each independently 0 to 4, preferably 0 or 1, more preferably 0. For example, R is a C₁₋₃ alkyl, each occurrence of R² and R³ is independently a hydrogen or a C₁₋₆ alkyl, and p and q are each independently 0 or 1. In another example, R is a methyl, each occurrence of R² and R³ is independently a hydrogen or a C₁₋₃ alkyl, and p and q are each 0 or 1.

The DPN compound can be a 4,4'-(((3-oxo-2-alkylisoindoline-1,1-diyl)bis(4,1-phenylene))bis(oxy)) diphthalonitrile of formula (1a). 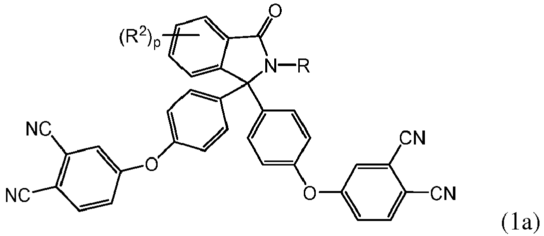 wherein R is a C₁₋₆ alkyl, more preferably a C₁₋₃ alkyl. For example, R is methyl. Each occurrence of R² is independently a hydrogen or C₁₋₆ alkyl, more preferably a hydrogen or C₁₋₃ alkyl. In formula (1a), p is independently 0 or 1, preferably 0. For example, R is a methyl, each occurrence of R² is independently a hydrogen or C₁₋₃ alkyl, and p is 0 or 1. In particular examples, the DPN compound is 4,4'-(((3-oxo-2-methylisoindoline-1,1-diyl)bis(4,1-phenylene))bis(oxy)) diphthalonitrile of formula (1b)

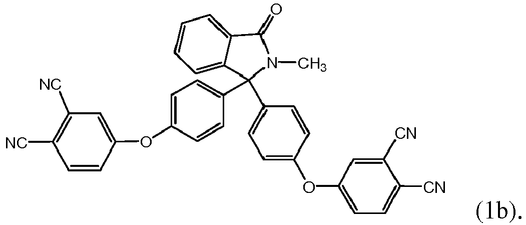

Also provided is a method for the manufacture of the 4,4'-(((3-oxo-2-hydrocarbylisoindoline-1,1-diyl)bis(4,1-arylene))bis(oxy)) diphthalonitrile compound (1). The method includes reacting a 3,3-bis(4-hydroxyaryl)-2-hydrocarbylisoindolin-1-one of formula (2) 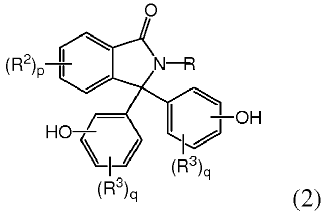 with 4-nitrophthalonitrile under conditions effective to provide the 4,4'-(((3-oxo-2-hydrocarbylisoindoline-1,1-diyl)bis(4,1-arylene))bis(oxy)) diphthalonitrile compound of formula (1).

In formula (2), R is a C₁₋₁₂ hydrocarbyl, preferably a C₁₋₆ alkyl. For example, R is methyl. Each occurrence of R² and R³ is independently a hydrogen, a halogen, a C₁₋₂₅ hydrocarbyl, preferably a hydrogen or a C₁₋₆ alkyl, more preferably a hydrogen or a C₁₋₃ alkyl. In formula (2), p and q are each independently 0 to 4, preferably 0 or 1, more preferably 0. In preferred examples, R is a C₁₋₃ alkyl, each occurrence of R² and R³ is independently a hydrogen or a C₁₋₆ alkyl, and p and q are each independently 0 or 1. For example, R is a methyl, each occurrence of R² and R³ is independently a hydrogen or a C₁₋₃ alkyl, and p and q are each 0 or 1.

For example, the 3,3-bis(4-hydroxyaryl)-2-hydrocarbylisoindolin-1-one (2) can be a 3,3-bis(4-hydroxyphenyl)-2-alkylisoindolin-1-one of formula (2a). 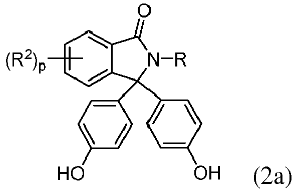 wherein R is a C₁₋₆ alkyl, more preferably a C₁₋₃ alkyl, such as methyl. Each occurrence of R² is independently a hydrogen or C₁₋₆ alkyl, more preferably a hydrogen or C₁₋₃ alkyl. In formula (2a), p is independently 0 or 1, preferably 0. For example, R is a methyl, each occurrence of R² is independently a hydrogen or C₁₋₃ alkyl, and p is 0 or 1. In particular examples, the 3,3-bis(4-hydroxyaryl)-2-hydrocarbylisoindolin-1-one (2) is 3,3-bis(4-hydroxyphenyl)-2-methylisoindolin-1-one (2) of formula (2b)

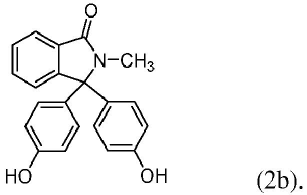

The reacting can be in a solvent in the presence of a inorganic base. Exemplary solvents include an alkyl pyrrolidone solvent, an aromatic solvent, a halogenated aromatic solvent, or combination thereof. For example, the solvent can be N-methyl-2-pyrrolidone, toluene, xylene, ortho-dichlorobenzene, 1,3-dimethyl-2-imidazolinone, 2-pyrrolidone, or a combination thereof. Exemplary inorganic bases include lithium carbonate, sodium carbonate, potassium carbonate, cesium carbonate, magnesium carbonate, calcium carbonate, or a combination thereof. For example, the inorganic base is potassium carbonate.

In a particular aspect, the method can include contacting an inorganic base and a 3,3-bis(4-hydroxyaryl)-2-arylisoindolin-1-one of formula (2) to provide a metal salt precursor; and reacting the metal salt precursor with 4-nitrophthalonitrile to provide the 4,4'-(((3-oxo-2-hydrocarbylisoindoline-1,1-diyl)bis(4,1-arylene))bis(oxy)) diphthalonitrile compound (1).

The reacting can further include the presence of a phase transfer catalyst. Examples of phase transfer catalysts include quaternary ammonium salts, quaternary phosphonium salts, sulphoxides, sulpholanes, guanidinium salts, imidazolium salts, or a combination thereof. Exemplary catalysts include tetramethylammonium chloride, tetramethylammonium bromide, trimethylbenzylammonium chloride, triethylbenzylammonium chloride, cetyltrimethylammonium bromide, methyltrioctylammonium chloride, trioctylbenzylammonium chloride, tributylbenzylammonium chloride , tetrabutylammonium bromide, tetraphenylphosphonium halides, methyltriphenylphosphonium iodide, ethyltriphenylphoshonium chloride, dimethylsulfoxide, diphenylsulfoxide, dibutylsulfoxide, sulfolane, 2,4-dimethylsulfolane, dimethylsulfone, diphenylsulfone, hexaethylguanidinium chloride, 1-butyl-3-methylimidazolium chloride, or a combination thereof. The phase transfer catalyst can be present in an amount of 0.5 to 80 mol%, based on the moles of the compound of formulas (2), (2a), or (2b).

Also provided is a thermosetting composition including the 4,4'-(((3-oxo-2-alkylisoindoline-1,1-diyl)bis(4,1-arylene))bis(oxy))diphthalonitrile compound of formula (1), (1a), or (1b) and an optional curing agent. The thermosetting composition can further include a comonomer, for example a diphthalonitrile different from formulas (1), (1a), and (1b). Exemplary diphthalonitriles include, but are not limited to, 3,3'-(1,3-phenylenedioxy)diphthalonitrile, 4,4'-[4,4'-biphenyldiylbis(oxy)]diphthalonitrile, (N-phenyl-2,2'-iminodiethanoxy)diphthalonitrile, [2,2'-(piperidine-1,4-diyldiethylene)di(tosylimino)]diphthalonitrile, or the like, or a combination thereof.

The diphthalonitrile monomers are generally solids at room or ambient temperatures, and can have melting points between 200 to 300°C. The polymerization of the diphthalonitrile monomers can be slow, and can require heating at elevated temperature for extended time before a vitrified product is obtained. Therefore, a curing agent can be used to promote the thermal polymerization reaction of the diphthalonitrile monomers. Exemplary curing agents include, but are not limited to, organic amines, phenols, strong organic acids and amine salts thereof, metals and salts thereof, or a combination thereof.

Preferably the curing agent is an amine-containing phosphazene, an aromatic diamine, an aromatic diamine/metal salt complex, an aromatic ether amine, an amine salt of p-toluene sulfonic acid, or an organic acid having an acid dissociation constant (pKa) in water of less than 3. More preferably, the curing agent can be an aromatic diamine or an organic acid having a pKa in water of less than 2. A combination of curing agents can also be used.

The curing agent can be a strong organic acid or combination of organic acids, each exhibiting pKa in water of less than 1.0. More preferably, a strong organic acid exhibits a pKₐ in water of less than 0.80. Strong organic acids include, for example, aromatic acids containing inorganic acidic substituents, such as the sulfonic acid group -SO₃H. Exemplary organic aromatic acids include p-toluenesulfonic acid, aniline-2-sulfonic acid, 8-aniline-1-naphthalenesulfonic acid, benzene sulfonic acid, butylsulfonic acid, 10-camphorsulfonic acid,2,5-diaminobenzenesulfonicacid,6-dimethylamino-4-hydroxy-2-naphthalenesulfonic acid, 5-dimethylamino-1-naphthalenesulfonic acid, 4-hydroxy-3-nitroso-1-naphthalenesulfonic acid tetrahydrate, 8-hydroxyquinoline-5-sulfonic acid, methylsulfonic acid, phenylboric acid, 1-naphthalenesulfonic acid, 2-naphthalenesulfonic acid, 1,5-naphthalenedisulfonic acid, 2,6-naphthalenedisulfonic acid, 2,7- napththalenedisulfonic acid, picrylsulfonic acid hydrate, 2-pyridineethanesulfonic acid, 4-pyridineethanesulfonic acid, 3-pyridinesulfonic acid, 2-pyridinylhydroxymethanesulfonic acid, sulfanilic acid, 2-sulfobenzoic acid hydrate, 5-sulfosalicylic acid hydrate, 2,4-xylenesulfonic acid, sulfonic acid containing dyes, or a combination thereof.

The curing agent can be an aromatic diamine such as a substituted or unsubstituted aromatic diamine of the formula (3)

Z(NH₂)₂ (3)

wherein Z is a C₆₋₂₂ arylene or a substituted derivative thereof. For example, the aromatic diamine can be o-phenylenediamine, m-phenylenediamine, p-phenylenediamine, 4,4'-diaminodiphenylpropane, 4,4'-diaminodiphenylmethane, 4,4'-diaminodiphenyl sulfide, 4,4'-diaminodiphenyl ether, 1,5-diaminonaphthalene, 3,3'-dimethylbenzidine, 3,3'-dimethoxybenzidine, 2,4-bis(β-amino-t-butyl)toluene, bis(p-β-amino-t-butyl)ether, bis(p-β-methyl-o-aminopentyl)benzene, 1,3-diamino-4-isopropylbenzene, 1,2-bis(3-aminopropoxy)ethane, benzidine, m-xylylenediamine, p-xylylenediamine, 2,4-diaminotoluene, 2,6-diaminotoluene, 1,3-bis(3-aminophenoxy)benzene, 1,3-bis(4-aminophenoxy)benzene, 1,4-bis(3-aminophenoxy)benzene, 1,4-bis(4-aminophenoxy)benzene, bis[4-(3-aminophenoxy)phenyl]sulfone, bis[4-(4-aminophenoxy)phenyl]sulfone, 4,4'-bis(3-aminophenoxy)biphenyl, 4,4'-bis(4-aminophenoxy)biphenyl, 2,2-bis[4-(3-aminophenoxy)phenyl]propane, 2,2-bis[4-(4-aminophenoxy)phenyl]propane, or a combination thereof.

The curing agent can also be a salt of any of the amines disclosed herein. Exemplary curing salts include bis(3-aminophenoxy)-1,3-benzene p-toluene sulfonate, p-phenylenediamine p-toluenesulfonate, bis(4-aminophenyl)methane hydrochloride, N-phenylbenzamidine p-toluene sulfonate, or a combination thereof.

The curing agent can be a metal salt complex of the diamines disclosed herein. Examples of metal salts include cuprous chloride, cuprous bromide, cuprous cyanide, cuprous ferricyanide, zinc chloride, zinc bromide, zinc iodide, zinc cyanide, zinc ferrocyanide, zinc acetate, zinc sulfide, silver chloride, ferrous chloride, ferric chloride, ferrous ferricyanide, ferrous chloroplatinate, ferrous fluoride, ferrous sulfate, cobaltous chloride, cobaltic sulfate, cobaltous cyanide, nickel chloride, nickel cyanide, nickel sulfate, nickel carbonate, stannic chloride, stannous chloride hydrate, a complex of triphenylphosphine oxide and stannous chloride, or a combination thereof.

The curing agent can be an aromatic ether amine. Aromatic ether amines include those described in U.S. Pat. No. 5,003,078, the entire content of which is incorporated herein by reference. Exemplary aromatic ether amines include 1,3-bis(3-aminophenoxy)benzene, 1,3-bis(4-aminophenoxy)benzene, 1,4-bis(3-aminophenoxy)benzene, 1,4-bis(4-aminophenoxy)benzene, 4,4'-bis(3-aminophenoxy)biphenyl, 4,4'-bis(4-aminophenoxy)biphenyl, 2,2-bis[4-(3-aminophenoxy)phenyl]propane, 2,2-bis[4-(4-aminophenoxy)phenyl]propane, or a combination thereof.

The curing agent can be an amine-containing phosphazene. Amine-containing phosphazenes include those described in U.S. Pat. No. 5,208,318, the entire content of which is incorporated herein by reference.

The curing agent can be included in the thermosetting composition in any effective amount. For example, the amount by weight of the curing agent can be 1 to 40 wt%, preferably 1 to 20 wt%, more preferably 5 to 10 wt%, based on the total weight of the diphthalonitrile compounds. In a particular example, the curing agent is an organic diamine or an organic acid as described above, and the amount of curing agent is 1 to 20 wt%, preferably 1 to 15 wt%, more preferably 5 to 10 wt%, based on the total weight of the diphthalonitrile compounds.

The thermosetting composition can further include an additive. The additive can be a particulate filler, fibrous filler, antioxidant, heat stabilizer, light stabilizer, ultraviolet light stabilizer, ultraviolet light-absorbing compound, near infrared light-absorbing compound, infrared light-absorbing compound, plasticizer, lubricant, release agent, antistatic agent, anti-fog agent, antimicrobial agent, colorant, surface effect additive, radiation stabilizer, flame retardant, anti-drip agent, fragrance, a polymer different from the thermoset polymer, or a combination thereof.

Examples of polymers different than the thermoset polymer include polyacetals (e.g., polyoxyethylene and polyoxymethylene), poly(C₁₋₆ alkyl)acrylates, polyacrylamides, polyamides, (e.g., aliphatic polyamides, polyphthalamides, and polyaramides), polyamideimides, polyanhydrides, polyarylates, polyarylene ethers (e.g., polyphenylene ethers), polyarylene sulfides (e.g., polyphenylene sulfides), polyarylene sulfones (e.g., polyphenylene sulfones), polybenzothiazoles, polybenzoxazoles, polycarbonates (including polycarbonate copolymers such as polycarbonate-siloxanes, polycarbonate-esters, and polycarbonate-ester-siloxanes), polyesters (e.g., polyethylene terephthalates, polybutylene terephthalates, polyarylates, and polyester copolymers such as polyester-ethers), polyetheretherketones, polyetherimides (including copolymers such as polyetherimide-siloxane copolymers), polyetherketoneketones, polyetherketones, polyethersulfones, polyimides (including copolymers such as polyimide-siloxane copolymers), poly(C₁₋₆ alkyl)methacrylates, polymethacrylamides, polynorbornenes (including copolymers containing norbornenyl units), polyolefins (e.g., polyethylenes, polypropylenes, polytetrafluoroethylenes, and their copolymers, for example ethylene-alphaolefin copolymers), polyoxadiazoles, polyoxymethylenes, polyphthalides, polysilazanes, polysiloxanes, polystyrenes (including copolymers such as acrylonitrile-butadiene-styrene (ABS) and methyl methacrylate-butadiene-styrene (MBS)), polysulfides, polysulfonamides, polysulfonates, polysulfones, polythioesters, polytriazines, polyureas, polyurethanes, polyvinyl alcohols, polyvinyl esters, polyvinyl ethers, polyvinyl halides, polyvinyl ketones, polyvinyl thioethers, polyvinylidene fluorides, or the like, or a combination thereof.

Also provide is a thermoset polymer composition comprising a crosslinked product of the thermosetting composition. The thermoset polymer composition can be prepared by any suitable method, including by polymerizing a 4,4'-(((3-oxo-2-alkylisoindoline-1,1-diyl)bis(4,1-arylene))bis(oxy))diphthalonitrile compound of formulas (1), (1a), and/or (1b) under conditions effective to form the crosslinked product. The thermoset polymer composition can further include an additive, including the additives described herein for the thermosetting composition.

The method of polymerizing includes heating the thermosetting compound. The heating can be at a temperature of 200 to 500°C, preferably 200 to 400°C, more preferably 200 to 300°C, even more preferably 220 to 300°C, optionally in the presence of a curing agent. The heating can further be at a pressure of 1 to 100 atmospheres (atm) (101.3 to 10132.5 kilopascals (kPa)), preferably 1 to 10 atm (101.3 to 1013.3 kPa), more preferably 1 to 5 atm (101.3 to 506.7 kPa). For example, the heating can be at a temperature of 200 to 500°C, preferably 200 to 400°C, more preferably 200 to 300°C, even more preferably 220 to 300°C and at a pressure of 1 to 100 atm (101.3 to 10132.5 kPa), preferably 1 to 10 atm (101.3 to 1013.3 kPa), more preferably 1 to 5 atm (101.3 to 506.7 kPa).

The heating can comprise a cure cycle. For example, the cure cycle can include a two-part cure of 225 to 260°C for 5 to 20 hours and 300 to 315°C for 5 to 20 hours; a three-part cure of 180 to 240°C for 2 to 16 hours, 240 to 300°C for 2 to 8 hours, and 300 to 315°C for 4 to 20 hours; or a four-part cure of 200 to 240°C for 1 to 3 hours, 240 to 270°C for 2 to 4 hours, 270 to 300°C for 4 to 6 hours, and 300 to 315°C for 4 to 20 hours.

Also provided is an article including the thermosetting composition or the thermoset polymer composition. The article can be in the form of a composite, a foam, a fiber, a layer, a coating, an encapsulant, an adhesive, a sealant, a component (e.g., a molded component), a prepreg, a casing, or a combination thereof. Specific examples of articles include is an electronic substrate, an electronic housing, a microelectronic device, a printed electronic component, a tooling, a geothermal tool, an oil rig component, a flame retardant component, a sizing resin, a resin infusion molded component, a resin transfer molded component, or a combination thereof. In still other example, the article is a carbon fiber composite, a fiberglass composite, a fiber coating, a weather resistant coating, a scratch resistant coating, a food packaging coating, an electrically insulative coating, a carbon fiber composite, a fiberglass composite, an industrial component, an oil rig component, a missile component, a rocket component, a building component, a bridge component, a transportation component, an electrical component, a computer component, an appliance component, a furniture component, a pump component, a electrolytic cell component, a tooling, a flame retardant component, a body protective component, a sporting equipment component, a container, a filament winding, a resin infusion molding component, a resin transfer molding component, or a combination thereof.

Exemplary articles include an exhaust stack, a scrubber, a stair case, a walkway, a tile, a vehicle, an aircraft or watercraft exterior component, submersible watercraft component, a floor pan, an air scoop, a pipe, a duct, a fan component, a fan housing, a blower, an industrial mixer component, a tile, a business machine component, or a pultruded part for structural applications. Other exemplary articles include a water craft hull, a watercraft deck, a marine terminal fender, a bridge deck, a bridge support, a railing, a building panel, a business machine housing or tray, a concrete modifier, a dishwasher or refrigerator part, an electrical encapsulant, an electrical panel, a electrorefining tank, a water softener tank, a fuel tank, a pressure tank, a filament-wound tank, a tank lining, a garage door, a grating, a protective body gear, a luggage component, a printed circuit board, an optical waveguide, a radome, a tank car, a hopper car cover, a car door, a truck bed liner, a satellite dish, a sign, a solar energy panel, a telephone switchgear housing, a tractor part, a transformer cover, a ground insulation, a turn insulation, a phase separation insulation for rotating machines, a commutator, a core insulation, a cord, a lacing tape, a drive shaft coupling, a propeller blade, a rocket motor case, a wing section, a sucker rod, a fuselage section, a wing skin wing fairing, an engine narcelle, a cargo door, a tennis racquet, a golf club shaft, a fishing rod, a ski, a ski pole, a bicycle part, a transverse leaf spring, an automotive smog pump component, a such as electrical cable joint, a wire winding, a sealant for an electromechanical device, a battery case, a resistor, a fuse, a thermal cut-off device, a capacitor, a transformer, a crankcase heater, a coil, a resistor, a semiconductor, a steel replacement component in chemical processing, a pulp and paper, a power generation, a and wastewater treatment, a scrubbing tower, a gratings, a safety rail, a component for a swimming pool, a swimming pool slide, a hot-tub, a or sauna, a drive shaft, a dry toner polymer for copying machine, a heat shield, a submarine hull, a laminated trim, a drilling fixture, a bonding jig, an inspection fixture, an industrial metal forming die, an aircraft stretch block and hammer form, a vacuum molding tool, a flooring, a antistatic articles, a decorative flooring; expansion joints for bridge, an injectable mortar for patch and repair of cracks in structural concrete, a grouting for tile, a machinery rail, a bolt and post, a storage tank repair, or a light emitting diode (LED) encapsulant.

Processes useful for preparing the articles and materials include those generally known to the art for the processing of thermosetting polymers. Such processes have been described in the literature as in, for example, Engineered Materials Handbook, Volume 1, Composites, ASM International Metals Park, Ohio, copyright 1987 Cyril A. Dostal Senior Ed, pp. 105-168 and 497-533, and "Polyesters and Their Applications" by Bjorksten Research Laboratories, Johan Bjorksten (pres.) Henry Tovey (Ch. Lit. Ass.), Betty Harker (Ad. Ass.), James Henning (Ad. Ass.), Reinhold Publishing Corporation, New York, 1956. Processing techniques include polymer transfer molding; sheet molding; bulk molding; pultrusion; injection molding, including reaction injection molding (RIM); atmospheric pressure molding (APM); casting, including centrifugal and static casting open mold casting; lamination including wet or dry lay up and spray lay up; also included are contact molding, including cylindrical contact molding; compression molding; including vacuum assisted polymer transfer molding and chemically assisted polymer transfer molding; matched tool molding; autoclave curing; thermal curing in air; vacuum bagging; pultrusion; Seeman's Composite Resin Infusion Manufacturing Processing (SCRIMP); open molding, continuous combination of polymer and glass; and filament winding, including cylindrical filament winding. For example, an article can be prepared from the disclosed thermoset polymer product via a polymer transfer molding process.

The compositions and methods described herein are further illustrated by the following non-limiting example.

### EXAMPLES

The materials listed in Table 1 were used.

**Table 1.**

| Component | Source |
|---|---|
| 3,3-bis(4-hydroxyphenyl)-2-methylisoindolin-1-one (2b) | SABIC |
| Potassium carbonate | Merck |
| N-methyl 2-pyrrolidone | Sigma-Aldrich |
| 4-nitrophthalonitrile | Sigma-Aldrich |

### Example 1. Preparation of 4,4'-(((3-oxo-2-methylisoindoline-1,1-diyl)bis(4,1-phenylene))bis(oxy)) diphthalonitrile (1b).

In a reaction vessel, 3,3-bis(4-hydroxyphenyl)-2-methylisoindolin-1-one (2b) (2.0 grams (g), 6.0 millimoles (mmol)) and potassium carbonate (2.95 g, 21.35 mmol) were stirred under a nitrogen atmosphere in N-methyl 2-pyrrolidone (50 mL) at 20 to 25°C for 30 minutes. After that, 4-nitrophthalonitrile (2.11 g, 12.2 mmol) was gradually added to the reaction mixture over a period of 10 minutes. The reaction mixture was maintained at 20 to 25°C for 24 hours with stirring. Progress of the reaction was monitored by thin-layer chromatography (TLC) [eluents: ethyl acetate (4 mL): hexanes (6 mL)]. Once the spot for compound (2b) disappeared by TLC, the resulting reaction mixture was filtered. After that, the obtained residue was added to a 50 wt% sodium chloride aqueous solution. A slightly yellowish colored solid crude product was obtained. Further, the crude product was purified with methanol. 3.22 g (91% yield) of off-white colored crystalline solid product was obtained therefrom. The product was subsequently dried under vacuum at 70°C for 16 hours.

The dried product was then analyzed using a Shimadzu high performance liquid chromatography (HPLC) system equipped with a quaternary solvent delivery system, an auto sampler, DAD detector, and a column compartment instrumentation that was controlled by ChemStation software. The column was a Poroshell 120, EC-C18, 4.6 mm x 100 mm x 2.7 µm. The mobile phase (A) was water and mobile phase (B) was acetonitrile. The gradient program used was 0-min B:20%, 10 min B:20%, 15 min B:90%, 22 min B:90 %, 23 min B:20%, and 30 min B:20%. The wavelength monitored was at 230 nm and the flow rate was 1.0 mL/min. The HPLC purity of the product (1b) was 99.67%.

Characterization by proton nuclear magnetic resonance (¹H NMR) (600 MHz, DMSO): chemical shift (δ, ppm) = 2.97 (triplet, 3H), 7.2 (dd, 4 H), 7.4 (d, 2 H), 7.5 (triplet, 2H), 7.6 (triplet, 1H), 7.75 (d, 1H), 7.8 (singlet, 2H), and 8.0 (d, 2H).

The melting point was 229.27°C, as determined by differential scanning calorimetry (DSC).

Liquid chromatography (LC-MS) analyses were conducted on a Waters triple quadruple Quattro micro mass spectrometer system, with the instrumentation controlled and synchronized by Mass Lync software. The instrument was operated with an electrospray ionization (EI) source in positive ion mode. The following are the LC-MS parameters used: Capillary voltage - 3.5 kilovolts (kV); Cone voltage 30 Volts (V); Extractor voltage 5 V; RF lens 0.1 V; Source temperature 1,300°C; Desolvation temperature: 3,500°C; dissolution gas flow rate 650 L/hr; Cone gas flow rate 30 L/hr. The product was confirmed by LC-MS as M/Z: 583.6; M+2 peak: 585.7; Molecular weight: 583.61 Daltons.

### Example 2. Preparation of 4,4'-(((3-oxo-2-methylisoindoline-1,1-diyl)bis(4,1-phenylene))bis(oxy)) diphthalonitrile (1b).

In a reaction vessel, 3,3-bis(4-hydroxyphenyl)-2-methylisoindolin-1-one (2b) (5.0 g, 15.0 mmol) and potassium carbonate (7.25 g, 30 mmol) were stirred under a nitrogen atmosphere with N-methyl 2-pyrrolidine (10 mL) and toluene (100 mL) at 20 to 25°C for 30 minutes. After that, the reaction mixture was heated at reflux for four hours. The reaction mixture was then cooled to 20 to 25°C, and 4-nitrophthalonitrile (2.11 g, 12.2 mmol) was gradually added over a period of 10 minutes. The reaction mixture was then heated at reflux for six hours with stirring. The progress of the reaction was monitored by TLC [eluents: ethyl acetate (4mL): hexanes (6 mL)] and HPLC. Once the signal for compound (2b) disappeared by TLC and was no longer detected by HPLC, the resulting reaction mixture was filtered. After that, the obtained solid was added to a 50 wt% aqueous sodium chloride solution. A slightly yellowish colored solid crude product was obtained. Further, the crude solid was purified with methanol to provide 7.98 g (∼91% yield) of an off-white colored crystalline solid product. The product was subsequently dried under vacuum at 70°C for 16 hours.

The purity of the product (1b) was measured by HPLC as described in Example 1. The observed purity was >99.60%.

Characterization by ¹H NMR (600 MHz, DMSO): δ (ppm) = 2.97 (triplet, 3H), 7.2 (dd, 4 H), 7.4 (d, 2 H), 7.5 (triplet, 2H), 7.6 (triplet, 1H), 7.75 (d, 1H), 7.8 (singlet, 2H), and 8.0 (d, 2H).

The melting point was 229.17°C (DSC).

The product was confirmed by LC-MS as described in Example 1, found M/Z: 583.6; M+2 peak: 585.7; Mol. wt: 583.61 Daltons.

The disclosure is further illustrated by the following non-limiting aspects.
Aspect 1. A thermosetting composition, comprising a 4,4'-(((3-oxo-2-hydrocarbylisoindoline-1,1-diyl)bis(4,1-arylene))bis(oxy)) diphthalonitrile compound of formula (1) wherein R is a C₁₋₁₂ hydrocarbyl, preferably a C₁₋₆ alkyl or a C₆₋₁₂ aryl; each occurrence of R² and R³ is independently a hydrogen, a halogen, or a C₁₋₂₅ hydrocarbyl, preferably hydrogen or a C₁₋₆ alkyl, more preferably a hydrogen or C₁₋₃ alkyl; and p and q are each independently 0-4, preferably 0 or 1, more preferably 0.
Aspect 2. The thermosetting composition of aspect 1, wherein the compound of formula (1) is a 4,4'-(((3-oxo-2-alkylisoindoline-1,1-diyl)bis(4,1-phenylene))bis(oxy)) diphthalonitrile of formula (1a) wherein R is a C₁₋₆ alkyl, more preferably a C₁₋₃ alkyl; each occurrence of R² is independently a hydrogen or C₁₋₆ alkyl, more preferably a hydrogen or C₁₋₃ alkyl; and p is independently 0 or 1, preferably 0.
Aspect 3. The thermosetting composition of aspect 1, wherein the compound of formula (1) is 4,4'-(((3-oxo-2-methylisoindoline-1,1-diyl)bis(4,1-phenylene))bis(oxy)) diphthalonitrile of formula (1b).
Aspect 4. The thermosetting composition of any one or more of the preceding aspects, further comprising a curing agent.
Aspect 5. The thermosetting composition of aspect 4, wherein the curing agent comprises a diamine, a triamine, a higher amine, an acid salt of an amine, or an organic acid having a pKa in water of less than 4, preferably an amine-containing phosphazene, an aromatic diamine, an aromatic diamine/metal salt complex, an aromatic ether amine, an amine salt of p-toluene sulfonic acid or an organic acid having a pKa in water of less than 3, more preferably an aromatic diamine or an organic acid having a pKa in water of less than 2.
Aspect 6. The thermosetting composition of aspect 5, wherein the aromatic diamine is o-phenylenediamine, m-phenylenediamine, p-phenylenediamine, 4,4'-diaminodiphenylpropane, 4,4'-diaminodiphenylmethane, 4,4'-diaminodiphenyl sulfide, 4,4'-diaminodiphenyl ether, 1,5-diaminonaphthalene, 3,3'-dimethylbenzidine, 3,3'-dimethoxybenzidine, 2,4-bis(β-amino-t-butyl)toluene, bis(p-β-amino-t-butyl)ether, bis(p-β-methyl-o-aminopentyl)benzene, 1,3-diamino-4-isopropylbenzene, 1,2-bis(3-aminopropoxy)ethane, benzidine, m-xylylenediamine, p-xylylenediamine, 2,4-diaminotoluene, 2,6-diaminotoluene, 1,3-bis(3-aminophenoxy)benzene, 1,3-bis(4-aminophenoxy)benzene, 1,4-bis(3-aminophenoxy)benzene, 1,4-bis(4-aminophenoxy)benzene, bis[4-(3-aminophenoxy)phenyl]sulfone, bis[4-(4-aminophenoxy)phenyl]sulfone, 4,4'-bis(3-aminophenoxy)biphenyl, 4,4'-bis(4-aminophenoxy)biphenyl, 2,2-bis[4-(3-aminophenoxy)phenyl]propane, 2,2-bis[4-(4-aminophenoxy)phenyl]propane, or a combination thereof.
Aspect 7. The thermosetting composition of any one or more of the preceding aspects, further comprising a comonomer, preferably wherein the comonomer is a diphthalonitrile different from formulas (1), (1a), and (1b).
Aspect 8. The thermosetting composition of any one or more of the preceding aspects, further comprising an additive, preferably wherein the additive is a particulate filler, fibrous filler, antioxidant, heat stabilizer, light stabilizer, ultraviolet light stabilizer, ultraviolet light-absorbing compound, near infrared light-absorbing compound, infrared light-absorbing compound, plasticizer, lubricant, release agent, antistatic agent, anti-fog agent, antimicrobial agent, colorant, surface effect additive, radiation stabilizer, flame retardant, anti-drip agent, fragrance, a polymer different from the thermoset polymer, or a combination thereof.
Aspect 9. A method for the manufacture of a 4,4'-(((3-oxo-2-hydrocarbylisoindoline-1,1-diyl)bis(4,1-arylene))bis(oxy))diphthalonitrile compound of formula (1), the method comprising reacting a 3,3-bis(4-hydroxyaryl)-2-hydrocarbylisoindolin-1-one of formula (2) with 4-nitrophthalonitrile under conditions effective to provide the 4,4'-(((3-oxo-2-hydrocarbylisoindoline-1,1-diyl)bis(4,1-arylene))bis(oxy)) diphthalonitrile compound (1), wherein R is a C₁₋₁₂ hydrocarbyl, preferably a C₁₋₆ alkyl; each occurrence of R² and R³ is independently a hydrogen, a halogen, a C₁₋₂₅ hydrocarbyl, preferably a hydrogen or a C₁₋₆ alkyl, more preferably a hydrogen or a C₁₋₃ alkyl; and p and q are each independently 0 to 4, preferably 0 or 1, more preferably 0.
Aspect 10. The method of aspect 9, wherein the 3,3-bis(4-hydroxyaryl)-2-hydrocarbylisoindolin-1-one (2) is a 3,3-bis(4-hydroxyphenyl)-2-alkylisoindolin-1-one of formula (2a) wherein R is a C₁₋₆ alkyl, more preferably a C₁₋₃ alkyl; each occurrence of R² is independently a hydrogen or C₁₋₆ alkyl, more preferably a hydrogen or C₁₋₃ alkyl; and p is independently 0 or 1, preferably 0; preferably wherein the 3,3-bis(4-hydroxyaryl)-2-alkylisoindolin-1-one (2a) is a 3,3-bis(4-hydroxyphenyl)-2-methylisoindolin-1-one of formula (2b).
Aspect 11. The method of any of aspects 9 or 10, wherein the reacting is in a solvent in the presence of an inorganic base, preferably wherein the solvent comprises an alkyl pyrrolidone solvent, an aromatic solvent, a halogenated aromatic solvent, or a combination thereof, more preferably the solvent comprises N-methyl-2-pyrrolidone, toluene, xylene, ortho-dichlorobenzene, 1,3-dimethyl-2-imidazolinone, 2-pyrrolidone, or a combination thereof, and preferably wherein the inorganic base comprises lithium carbonate, sodium carbonate, potassium carbonate, cesium carbonate, magnesium carbonate, calcium carbonate, or a combination thereof, more preferably the inorganic base comprises potassium carbonate.
Aspect 12. The method of any one or more of aspects 9 to 11, further comprising contacting an inorganic base and a 3,3-bis(4-hydroxyaryl)-2-arylisoindolin-1-one of formula (2) to provide a metal salt precursor; and reacting the metal salt precursor with 4-nitrophthalonitrile.
Aspect 13. A thermoset polymer composition comprising a crosslinked product of the thermosetting composition of any one or more of aspects 1 to 8.
Aspect 14. An article comprising the thermoset polymer composition of aspect 13, wherein the article is a composite, a foam, a fiber, a layer, a coating, an encapsulant, an adhesive, a sealant, a component, a prepreg, a casing, or a combination thereof.
Aspect 15. The article of aspect 14, wherein the article is an electronic substrate, an electronic housing, a microelectronic device, a printed electronic component, a tooling, a geothermal tool, an oil rig component, a flame retardant component, a sizing resin, a resin infusion molded component, a resin transfer molded component, or a combination thereof.

The compositions, methods, and articles can alternatively comprise, consist of, or consist essentially of, any appropriate materials, steps, or components herein disclosed. The compositions, methods, and articles can additionally, or alternatively, be formulated so as to be devoid, or substantially free, of any materials (or species), steps, or components, that are otherwise not necessary to the achievement of the function or objectives of the compositions, methods, and articles.

All ranges disclosed herein are inclusive of the endpoints, and the endpoints are independently combinable with each other (e.g., ranges of "up to 25 wt%, or, more specifically, 5-20 wt%", is inclusive of the endpoints and all intermediate values of the ranges of "5 wt% to 25 wt%," etc.). "Combinations" is inclusive of blends, mixtures, alloys, reaction products, and the like. The terms "a" and "an" and "the" do not denote a limitation of quantity, and are to be construed to cover both the singular and the plural, unless otherwise indicated herein or clearly contradicted by context. "Or" means "and/or" unless clearly stated otherwise. Reference throughout the specification to "some embodiments", "an embodiment", "an example" and so forth, means that a particular element described in connection with the embodiment is included in at least one embodiment or example described herein, and may or may not be present in other embodiments. In addition, it is to be understood that the described elements can be combined in any suitable manner in the various embodiments. "Combination thereof' is an open term that includes one or more of the named elements, optionally together with a like element not named.

Unless specified to the contrary herein, all test standards are the most recent standard in effect as of the filing date of this application, or, if priority is claimed, the filing date of the earliest priority application in which the test standard appears.

Unless defined otherwise, technical and scientific terms used herein have the same meaning as is commonly understood by one of skill in the art to which this application belongs. All cited patents, patent applications, and other references are incorporated herein by reference in their entirety. However, if a term in the present application contradicts or conflicts with a term in the incorporated reference, the term from the present application takes precedence over the conflicting term from the incorporated reference.

The term "hydrocarbyl" and "hydrocarbon" refers broadly to a group comprising carbon and hydrogen, optionally with 1 to 3 heteroatoms, for example, oxygen, nitrogen, halogen, silicon, sulfur, or a combination thereof. The term "aliphatic" means a branched or unbranched, saturated or unsaturated group containing carbon and hydrogen, optionally with 1 to 3 heteroatoms, for example, oxygen, nitrogen, halogen, silicon, sulfur, or a combination thereof. The term "cycloaliphatic" means a saturated or unsaturated group comprising carbon and hydrogen optionally with 1 to 3 heteroatoms, for example, oxygen, nitrogen, halogen, silicon, sulfur, or a combination thereof. The term "alkyl" means a branched or straight chain, unsaturated aliphatic hydrocarbon group, e.g., methyl, ethyl, n-propyl, i-propyl, n-butyl, s-butyl, t-butyl, n-pentyl, s-pentyl, and n- and s-hexyl. "Alkenyl" means a straight or branched chain, monovalent hydrocarbon group having at least one carbon-carbon double bond (e.g., ethenyl (-HC=CH₂)). "Alkoxy" means an alkyl group that is linked via an oxygen (i.e., alkyl-O-), for example methoxy, ethoxy, and sec-butyloxy groups. "Alkylene" means a straight or branched chain, saturated, divalent aliphatic hydrocarbon group (e.g., methylene (-CH₂-) or, propylene (-(CH₂)₃-)). "Cycloalkylene" means a divalent cyclic alkylene group, -CₙH₂ₙ₋ₓ, wherein x is the number of hydrogens replaced by cyclization(s). "Cycloalkenyl" means a monovalent group having one or more rings and one or more carbon-carbon double bonds in the ring, wherein all ring members are carbon (e.g., cyclopentyl and cyclohexyl). "Aryl" means an aromatic hydrocarbon group containing the specified number of carbon atoms, such as phenyl, tropone, indanyl, or naphthyl. "Arylene" means a divalent aryl group. "Alkylarylene" means an arylene group substituted with an alkyl group. "Arylalkylene" means an alkylene group substituted with an aryl group (e.g., benzyl). The prefix "halo" means a group or compound including one more of a fluoro, chloro, bromo, or iodo substituent. A combination of different halo groups (e.g., bromo and fluoro), or only chloro groups can be present. The prefix "hetero" means that the compound or group includes at least one ring member that is a heteroatom (e.g., 1, 2, or 3 heteroatom(s)), wherein the heteroatom(s) is each independently N, O, S, Si, or P.

Unless otherwise indicated, each of the foregoing groups can be unsubstituted or substituted, provided that the substitution does not significantly adversely affect synthesis, stability, or use of the compound. "Substituted" means that the compound, group, or atom is substituted with at least one (e.g., 1, 2, 3, or 4) substituents instead of hydrogen, where each substituent is independently nitro (-NO₂), cyano (-CN), hydroxy (-OH), halogen, thiol (-SH), thiocyano (-SCN), C₁₋₆ alkyl, C₂₋₆ alkenyl, C₂₋₆ alkynyl, C₁₋₆ haloalkyl, C₁₋₉ alkoxy, C₁₋₆ haloalkoxy, C₃₋₁₂ cycloalkyl, C₅₋₁₈ cycloalkenyl, C₆₋₁₂ aryl, C₇₋₁₃ arylalkylene (e.g., benzyl), C₇₋₁₂ alkylarylene (e.g., toluyl), C₄₋₁₂ heterocycloalkyl, C₃₋₁₂ heteroaryl, C₁₋₆ alkyl sulfonyl (-S(=O)₂-alkyl), C₆₋₁₂ arylsulfonyl (-S(=O)₂-aryl), or tosyl (CH₃C₆H₄SO₂-), provided that the substituted atom's normal valence is not exceeded, and that the substitution does not significantly adversely affect the manufacture, stability, or desired property of the compound. When a compound is substituted, the indicated number of carbon atoms is the total number of carbon atoms in the compound or group, including those of any substituents.

While particular embodiments have been described, alternatives, modifications, variations, improvements, and substantial equivalents that are or may be presently unforeseen may arise to applicants or others skilled in the art. Accordingly, the appended claims as filed and as they may be amended are intended to embrace all such alternatives, modifications variations, improvements, and substantial equivalents.

## Claims

1. A thermosetting composition, comprising a 4,4'-(((3-oxo-2-hydrocarbylisoindoline-1,1-diyl)bis(4,1-arylene))bis(oxy)) diphthalonitrile compound of formula (1) 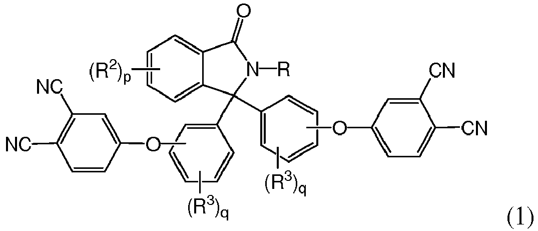 wherein
R is a C₁₋₁₂ hydrocarbyl, preferably a C₁₋₆ alkyl or a C₆₋₁₂ aryl;
each occurrence of R² and R³ is independently a hydrogen, a halogen, or a C₁₋₂₅ hydrocarbyl, preferably hydrogen or a C₁₋₆ alkyl, more preferably a hydrogen or C₁₋₃ alkyl; and
p and q are each independently 0 to 4, preferably 0 or 1, more preferably 0.

2. The thermosetting composition of claim 1, wherein the compound is a 4,4'-(((3-oxo-2-alkylisoindoline-1,1-diyl)bis(4,1-phenylene))bis(oxy)) diphthalonitrile of formula (1a) 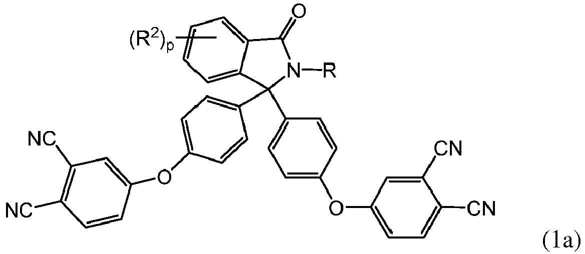 wherein
R is a C₁₋₆ alkyl or a C₆₋₁₂ aryl, more preferably a C₁₋₃ alkyl or phenyl;
each occurrence of R² is independently a hydrogen or C₁₋₆ alkyl, more preferably a hydrogen or C₁₋₃ alkyl; and
p is independently 0 or 1, preferably 0.

3. The thermosetting composition of claim 1, wherein the compound of formula (1) is 4,4'-(((3-oxo-2-methylisoindoline-1,1-diyl)bis(4,1-phenylene))bis(oxy))diphthalonitrile of formula (1b)

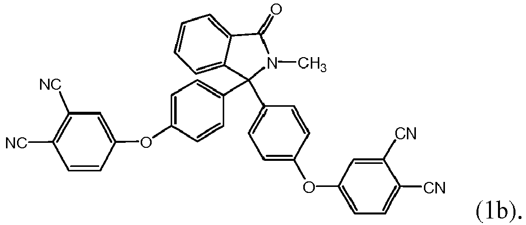

4. The thermosetting composition of any one or more of the preceding claims, further comprising a curing agent.

5. The thermosetting composition of claim 4, wherein the curing agent comprises a diamine, a triamine, a higher amine, an acid salt of an amine, or an organic acid having a pKa in water of less than 4, preferably an amine-containing phosphazene, an aromatic diamine, an aromatic diamine/metal salt complex, an aromatic ether amine, an amine salt of p-toluene sulfonic acid or an organic acid having a pKa in water of less than 3, more preferably an aromatic diamine or an organic acid having a pKa in water of less than 2.

6. The thermosetting composition of claim 5, wherein the aromatic diamine is o-phenylenediamine, m-phenylenediamine, p-phenylenediamine, 4,4'-diaminodiphenylpropane, 4,4'-diaminodiphenylmethane, 4,4'-diaminodiphenyl sulfide, 4,4'-diaminodiphenyl ether, 1,5-diaminonaphthalene, 3,3'-dimethylbenzidine, 3,3'-dimethoxybenzidine, 2,4-bis(β-amino-t-butyl)toluene, bis(p-β-amino-t-butyl)ether, bis(p-β-methyl-o-aminopentyl)benzene, 1,3-diamino-4-isopropylbenzene, 1,2-bis(3-aminopropoxy)ethane, benzidine, m-xylylenediamine, p-xylylenediamine, 2,4-diaminotoluene, 2,6-diaminotoluene, 1,3-bis(3-aminophenoxy)benzene, 1,3-bis(4-aminophenoxy)benzene, 1,4-bis(3-aminophenoxy)benzene, 1,4-bis(4-aminophenoxy)benzene, bis[4-(3-aminophenoxy)phenyl]sulfone, bis[4-(4-aminophenoxy)phenyl]sulfone, 4,4'-bis(3-aminophenoxy)biphenyl, 4,4'-bis(4-aminophenoxy)biphenyl, 2,2-bis[4-(3-aminophenoxy)phenyl]propane, 2,2-bis[4-(4-aminophenoxy)phenyl]propane, or a combination thereof.

7. The thermosetting composition of any one or more of the preceding claims, further comprising a comonomer, preferably wherein the comonomer is a diphthalonitrile different from formulas (1), (1a), and (1b).

8. The thermosetting composition of any one or more of the preceding claims, further comprising an additive, preferably wherein the additive is a particulate filler, fibrous filler, antioxidant, heat stabilizer, light stabilizer, ultraviolet light stabilizer, ultraviolet light-absorbing compound, near infrared light-absorbing compound, infrared light-absorbing compound, plasticizer, lubricant, release agent, antistatic agent, anti-fog agent, antimicrobial agent, colorant, surface effect additive, radiation stabilizer, flame retardant, anti-drip agent, fragrance, a polymer different from the thermoset polymer, or a combination thereof.

9. A method for the manufacture of a 4,4'-(((3-oxo-2-hydrocarbylisoindoline-1,1-diyl)bis(4,1-arylene))bis(oxy)) diphthalonitrile compound of formula (1), 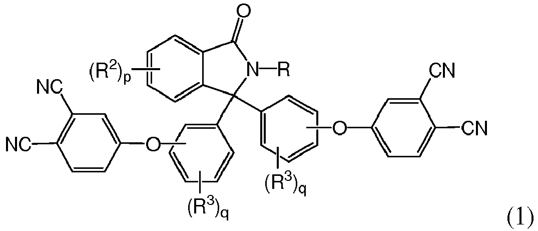 the method comprising:
reacting a 3,3-bis(4-hydroxyaryl)-2-hydrocarbylisoindolin-1-one of formula (2) 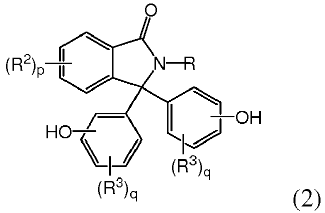 with 4-nitrophthalonitrile under conditions effective to provide the 4,4'-(((3-oxo-2-hydrocarbylisoindoline-1,1-diyl)bis(4,1-arylene))bis(oxy)) diphthalonitrile compound (1), whe
rein
R is a C₁₋₁₂ hydrocarbyl, preferably a C₁₋₆ alkyl;
each occurrence of R² and R³ is independently a hydrogen, a halogen, a C₁₋₂₅ hydrocarbyl, preferably a hydrogen or a C₁₋₆ alkyl, more preferably a hydrogen or a C₁₋₃ alkyl; and
p and q are each independently 0 to 4, preferably 0 or 1, more preferably 0.

10. The method of claim 9, wherein the 3,3-bis(4-hydroxyaryl)-2-hydrocarbylisoindolin-1-one (2) is a 3,3-bis(4-hydroxyphenyl)-2-alkylisoindolin-1-one of formula (2a) 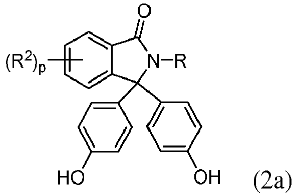 wherein R is a C₁₋₆ alkyl, more preferably a C₁₋₃ alkyl; each occurrence of R² is independently a hydrogen or C₁₋₆ alkyl, more preferably a hydrogen or C₁₋₃ alkyl; and p is independently 0 or 1, preferably 0;
preferably wherein the 3,3-bis(4-hydroxyaryl)-2-alkylisoindolin-1-one (2) is a 3,3-bis(4-hydroxyphenyl)-2-methylisoindolin-1-one of formula (2b)

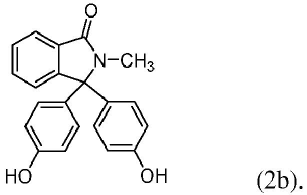

11. The method of any of claims 9 or 10, wherein the reacting is in a solvent in the presence of an inorganic base,
preferably wherein the solvent comprises an alkyl pyrrolidone solvent, an aromatic solvent, a halogenated aromatic solvent, or a combination thereof, more preferably the solvent comprises N-methyl-2-pyrrolidone, toluene, xylene, ortho-dichlorobenzene, 1,3-dimethyl-2-imidazolinone, 2-pyrrolidone, or a combination thereof, and
preferably wherein the inorganic base comprises lithium carbonate, sodium carbonate, potassium carbonate, cesium carbonate, magnesium carbonate, calcium carbonate, or a combination thereof, more preferably the inorganic base is potassium carbonate.

12. The method of any one or more of claims 9 to 11, further comprising contacting an inorganic base and a 3,3-bis(4-hydroxyaryl)-2-arylisoindolin-1-one of formula (2) to provide a metal salt precursor; and reacting the metal salt precursor with 4-nitrophthalonitrile.

13. A thermoset polymer composition comprising a crosslinked product of the thermosetting composition of any one or more of claims 1 to 8.

14. An article comprising the thermoset polymer composition of claim 13, wherein the article is a composite, a foam, a fiber, a layer, a coating, an encapsulant, an adhesive, a sealant, a component, a prepreg, a casing, or a combination thereof.

15. The article of claim 14, wherein the article is an electronic substrate, an electronic housing, a microelectronic device, a printed electronic component, a tooling, a geothermal tool, an oil rig component, a flame retardant component, a sizing resin, a resin infusion molded component, a resin transfer molded component, or a combination thereof.
